# EUROPÄISCHE PATENTANMELDUNG

(11) **EP 2 258 875 A1**
(43) Veröffentlichungstag der Anmeldung: **08.12.2010**
(21) Anmeldenummer: 10160616.8
(22) Anmeldetag: 21.04.2010
(51) Int. Cl.: C12Q 1/68

(54) **Verfahren zum Nachweis von Mykorrhizapilzen**

(30) Priorität: 21.04.2009 DE 102009017965
(71) Anmelder: Leibniz-Institut für Pflanzengenetik und Kulturpflanzenforschung (IPK), 06466 Gatersleben (DE); AMykor GmbH, 06803 Greppin (DE)
(72) Erfinder: Florschütz, Kristina, 06484, Quedlinburg (DE); Watzke, Katja, 06792, Sandersdorf (DE); Watzke, Roland, 06842, Dessau-Roßlau (DE); Kunze, Gotthard, 06466, Gatersleben (DE); Hacker, Rainer, 06749, Bitterfeld-Wolfen (DE)
(74) Vertreter: Taruttis, Stefan Georg

(57) **Zusammenfassung**

Die Erfindung betrifft ein Verfahren zur Analyse einer Probe, die Erde und/oder pflanzliches Wurzelgewebe enthalten kann, auf den Gehalt an Mykorrhizapilzen, vorzugsweise arbuskulären Mykorrhizapilzen, besonders bevorzugt *Glomus intraradices,* durch unmittelbare Kontaktierung von Gesamt-DNA, die aus der Probe isoliert wurde und wahlweise Hemmstoffe der PCR enthalten kann, durch Hybridisierung mit zumindest einem einzelsträngigen Oligonukleotid, das auf einem Träger immobilisiert ist, mit anschließender Dctcktion der Hybridisicrung.

SEQUENCE LISTING

<110> Leibniz-Institut für Pflanzengenetik und
Kulturpflanzenforschung
AMykor GmbH

<120> Verfahren zum Nachweis von Mykorrhizapilzen

<130> I1007EP

<160> 81

<170> PatentIn version 3.5

<210> 1
<211> 18
<212> DNA
<213> Artificial Sequence

<220>
<223> /note="Nukleinsäuresonde Seq.-ID Nr. 1"

<400> 1
atgagcgaca caaaccag 18

<210> 2
<211> 21
<212> DNA
<213> Artificial Sequence

<220>
<223> /note="Nukleinsäuresonde Seq.-ID Nr. 2"

<400> 2
cgaatacgat tcatcagatt t 21

<210> 3
<211> 20
<212> DNA
<213> Artificial Sequence

<220>
<223> /note="Nukleinsäuresonde Seq.-ID Nr. 3"

<400> 3
atgtttgtga gcttctttct 20

<210> 4
<211> 19
<212> DNA
<213> Artificial Sequence

<220>
<223> /note="Nukleinsäuresonde Seq.-ID Nr. 4"

<400> 4
atgtctgaac catccagag 19

<210> 5
<211> 22
<212> DNA
<213> Artificial Sequence

<220>
<223> /note="Nukleinsäuresonde Seq.-ID Nr. 5"

<400>  5
atgtgcaaac atatacttaa cg                                                22

<210>  6
<211>  22
<212>  DNA
<213>  Artificial Sequence

<220>
<223>  /note="Nukleinsäuresonde Seq.-ID Nr. 6"

<400>  6
aagtcttgat tgagaatact ct                                                22

<210>  7
<211>  20
<212>  DNA
<213>  Artificial Sequence

<220>
<223>  /note="Nukleinsäuresonde Seq.-ID Nr. 7"

<400>  7
atgggagatt ctatagaaag                                                   20

<210>  8
<211>  19
<212>  DNA
<213>  Artificial Sequence

<220>
<223>  /note="Nukleinsäuresonde Seq.-ID Nr. 8"

<400>  8
cctttagtac caaggaaga                                                    19

<210>  9
<211>  19
<212>  DNA
<213>  Artificial Sequence

<220>
<223>  /note="Nukleinsäuresonde Seq.-ID Nr. 9"

<400>  9
gtgtttacac cccttgaaa                                                    19

<210>  10
<211>  21
<212>  DNA
<213>  Artificial Sequence

<220>
<223>  /note="Nukleinsäuresonde Seq.-ID Nr. 10"

<400>  10
aagttgatca ggttgaatat a                                                 21

<210>  11
<211>  22
<212>  DNA
<213>  Artificial Sequence

<220>
<223>  /note="Nukleinsäuresonde Seq.-ID Nr. 11"

<400>  11
atgaatggga taataatttt at                                                22


<210>  12
<211>  17
<212>  DNA
<213>  Artificial Sequence

<220>
<223>  /note="Nukleinsäuresonde Seq.-ID Nr. 12"

<400>  12
gggttgaaag tggtgcc                                                      17


<210>  13
<211>  20
<212>  DNA
<213>  Artificial Sequence

<220>
<223>  /note="Nukleinsäuresonde Seq.-ID Nr. 13"

<400>  13
atgggagatt ctatagaaag                                                   20


<210>  14
<211>  18
<212>  DNA
<213>  Artificial Sequence

<220>
<223>  /note="Nukleinsäuresonde Seq.-ID Nr. 14"

<400>  14
aatttggtcc acccgaag                                                     18


<210>  15
<211>  21
<212>  DNA
<213>  Artificial Sequence

<220>
<223>  /note="Nukleinsäuresonde Seq.-ID Nr. 15"

<400>  15
atgtggaaaa tgaagatcat t                                                 21


<210>  16
<211>  18
<212>  DNA
<213>  Artificial Sequence

<220>
<223>  /note="Nukleinsäuresonde Seq.-ID Nr. 16"

<400>  16
aataactggt ccaccagg                                                     18


<210>  17

<211>  19
<212>  DNA
<213>  Artificial Sequence

<220>
<223>  /note="Nukleinsäuresonde Seq.-ID Nr. 17"

<400>  17
gtggtatcaa cgcagagca                                                    19


<210>  18
<211>  16
<212>  DNA
<213>  Artificial Sequence

<220>
<223>  /note="Nukleinsäuresonde Seq.-ID Nr. 18"

<400>  18
gcgtggtcgc ggccga                                                       16


<210>  19
<211>  21
<212>  DNA
<213>  Artificial Sequence

<220>
<223>  /note="Nukleinsäuresonde Seq.-ID Nr. 19"

<400>  19
gtgccattta gtaaaacagg a                                                 21


<210>  20
<211>  19
<212>  DNA
<213>  Artificial Sequence

<220>
<223>  /note="Nukleinsäuresonde Seq.-ID Nr. 20"

<400>  20
ccagcagcac caatgataa                                                    19


<210>  21
<211>  20
<212>  DNA
<213>  Artificial Sequence

<220>
<223>  /note="Nukleinsäuresonde Seq.-ID Nr. 21"

<400>  21
atgttgctca gactgttagc                                                   20


<210>  22
<211>  16
<212>  DNA
<213>  Artificial Sequence

<220>
<223>  /note="Nukleinsäuresonde Seq.-ID Nr. 22"

<400>  22
gcgtggtcgc ggccga                                                       16

<210>  23
<211>  21
<212>  DNA
<213>  Artificial Sequence

<220>
<223>  /note="Nukleinsäuresonde Seq.-ID Nr. 23"

<400>  23
gtggagttaa acttacacgt t                                                21


<210>  24
<211>  22
<212>  DNA
<213>  Artificial Sequence

<220>
<223>  /note="Nukleinsäuresonde Seq.-ID Nr. 24"

<400>  24
aaaaacatct ctagttggtt ct                                               22


<210>  25
<211>  23
<212>  DNA
<213>  Artificial Sequence

<220>
<223>  /note="Nukleinsäuresonde Seq.-ID Nr. 25"

<400>  25
atggcattat taaatttgac gaa                                              23


<210>  26
<211>  20
<212>  DNA
<213>  Artificial Sequence

<220>
<223>  /note="Nukleinsäuresonde Seq.-ID Nr. 26"

<400>  26
caggtttaaa cgaattcgcc                                                  20


<210>  27
<211>  18
<212>  DNA
<213>  Artificial Sequence

<220>
<223>  /note="Nukleinsäuresonde Seq.-ID Nr. 27"

<400>  27
atggacgtgg aagcgaga                                                    18


<210>  28
<211>  19
<212>  DNA
<213>  Artificial Sequence

<220>
<223>  /note="Nukleinsäuresonde Seq.-ID Nr. 28"

<400>  28
gcgtggtcgc ggcggccga                                                    19


<210>  29
<211>  18
<212>  DNA
<213>  Artificial Sequence

<220>
<223>  /note="Nukleinsäuresonde Seq.-ID Nr. 29"

<400>  29
   atggtacgcg ggggacaa                                                      18


<210>  30
<211>  21
<212>  DNA
<213>  Artificial Sequence

<220>
<223>  /note="Nukleinsäuresonde Seq.-ID Nr. 30"

<400>  30
ttattacata ttgactgtcg g                                                 21


<210>  31
<211>  20
<212>  DNA
<213>  Artificial Sequence

<220>
<223>  /note="Nukleinsäuresonde Seq.-ID Nr. 31"

<400>  31
acatctactt ccctatcagt                                                   20


<210>  32
<211>  19
<212>  DNA
<213>  Artificial Sequence

<220>
<223>  /note="Nukleinsäuresonde Seq.-ID Nr. 32"

<400>  32
tggaaccact ggattgtga                                                    19


<210>  33
<211>  20
<212>  DNA
<213>  Artificial Sequence

<220>
<223>  /note="Nukleinsäuresonde Seq.-ID Nr. 33"

<400>  33
agtttgcgta gatattgaag                                                   20


<210>  34
<211>  20
<212>  DNA
<213>  Artificial Sequence

<220>
<223>  /note="Nukleinsäuresonde Seq.-ID Nr. 34"

<400>  34
taacaaaact cgacctgtcc                                                    20


<210>  35
<211>  22
<212>  DNA
<213>  Artificial Sequence

<220>
<223>  /note="Nukleinsäuresonde Seq.-ID Nr. 35"

<400>  35
ttcatcagtt agattattac ca                                                 22


<210>  36
<211>  20
<212>  DNA
<213>  Artificial Sequence

<220>
<223>  /note="Nukleinsäuresonde Seq.-ID Nr. 36"

<400>  36
ctttccatct cataaggatg                                                    20


<210>  37
<211>  23
<212>  DNA
<213>  Artificial Sequence

<220>
<223>  /note="Nukleinsäuresonde Seq.-ID Nr. 37"

<400>  37
atgtataaca ctaataagtc aaa                                                23


<210>  38
<211>  19
<212>  DNA
<213>  Artificial Sequence

<220>
<223>  /note="Nukleinsäuresonde Seq.-ID Nr. 38"

<400>  38
gcgcatgaat gacgatgat                                                     19


<210>  39
<211>  21
<212>  DNA
<213>  Artificial Sequence

<220>
<223>  /note="Nukleinsäuresonde Seq.-ID Nr. 39"

<400>  39
aacaataatt tgagtgacac g                                                  21


<210>  40

<211>  22
<212>  DNA
<213>  Artificial Sequence

<220>
<223>  /note="Nukleinsäuresonde Seq.-ID Nr. 40"

<400>  40
atataaggag gtttgaaaat tt                                                22


<210>  41
<211>  22
<212>  DNA
<213>  Artificial Sequence

<220>
<223>  /note="Nukleinsäuresonde Seq.-ID Nr. 41"

<400>  41
atgttacctt tattctcaag ta                                                22


<210>  42
<211>  21
<212>  DNA
<213>  Artificial Sequence

<220>
<223>  /note="Nukleinsäuresonde Seq.-ID Nr. 42"

<400>  42
aagtatttac agtttcagta g                                                 21


<210>  43
<211>  18
<212>  DNA
<213>  Artificial Sequence

<220>
<223>  /note="Nukleinsäuresonde Seq.-ID Nr. 43"

<400>  43
attctaagcc ctcggcgt                                                     18


<210>  44
<211>  21
<212>  DNA
<213>  Artificial Sequence

<220>
<223>  /note="Nukleinsäuresonde Seq.-ID Nr. 44"

<400>  44
actccaatga cttaccttaa t                                                 21


<210>  45
<211>  17
<212>  DNA
<213>  Artificial Sequence

<220>
<223>  /note="Nukleinsäuresonde Seq.-ID Nr. 45"

<400>  45
cttagcgtgg tcgcggc                                                      17

<210>  46
<211>  24
<212>  DNA
<213>  Artificial Sequence

<220>
<223>  /note="Nukleinsäuresonde Seq.-ID Nr. 46"

<400>  46
tattcattaa taatgaattc gttg                                              24


<210>  47
<211>  20
<212>  DNA
<213>  Artificial Sequence

<220>
<223>  /note="Nukleinsäuresonde Seq.-ID Nr. 47"

<400>  47
tatgagccca tttaactgct                                                   20


<210>  48
<211>  18
<212>  DNA
<213>  Artificial Sequence

<220>
<223>  /note="Nukleinsäuresonde Seq.-ID Nr. 48"

<400>  48
gcaggtactt cgcgaagt                                                     18


<210>  49
<211>  21
<212>  DNA
<213>  Artificial Sequence

<220>
<223>  /note="Nukleinsäuresonde Seq.-ID Nr. 49"

<400>  49
cctggattat gctttatcta t                                                 21


<210>  50
<211>  19
<212>  DNA
<213>  Artificial Sequence

<220>
<223>  /note="Nukleinsäuresonde Seq.-ID Nr. 50"

<400>  50
atcctggtca tggaatgag                                                    19


<210>  51
<211>  21
<212>  DNA
<213>  Artificial Sequence

<220>
<223>  /note="Nukleinsäuresonde Seq.-ID Nr. 51"

<400>  51
tacatgtcca tcaagttctt t                                                21

<210>  52
<211>  22
<212>  DNA
<213>  Artificial Sequence

<220>
<223>  /note="Nukleinsäuresonde Seq.-ID Nr. 52"

<400>  52
atggctagtc aaattattgt cg                                               22

<210>  53
<211>  21
<212>  DNA
<213>  Artificial Sequence

<220>
<223>  </note="Nukleinsäuresonde Seq.-ID Nr. 53"

<400>  53
ggcaactgac attaatctca t                                                21

<210>  54
<211>  21
<212>  DNA
<213>  Artificial Sequence

<220>
<223>  /note="Nukleinsäuresonde Seq.-ID Nr. 54"

<400>  54
atgctcttga agcaaagaac a                                                21

<210>  55
<211>  689
<212>  DNA
<213>  Artificial Sequence

<220>
<223>  /note="Nukleinsäuresonde Seq.-ID Nr. 55"

<400>  55
gggggactgg tatacgactc actatagggc gaattgaatt tagcggccgc gaattcgccc      60

ttaagcagtg gtatcaccgt agagtacttt tttttttttt tttttttttt tttttttggg     120

catagacgaa tacgattcat cagatttttc gtgaaaaagc aacaactgaa atgttggttt     180

tggatccgta aaagttagtt tgtgaggaac ttcataatga gatttttgga aattaagatc      240

atgaaaaaaa gtaaaaggaa taatggtttt atctgtaaaa tataatgtta tcttcatatc     300

aaactcgccc caaccttttt cagaaagtgt aaatggaggt ttccgcacca ctctttcagg     360

aggttcaaat gttggatgta agatatattc aactctttca acgtaattta gaggacaatg     420

ttctcccata tcgtcaattg caaagatcga aaccttccac ttacgcattg gaagaccatt     480

agttggcttc gaatgctttg atatctcctg gtcggttaca actacaatct ctttttcaac     540

tattgactgg tttgtgtcgc tcatatttgg agaggtacga aagttgaaat aacttgctaa     600

tttttattaa agaatctgtg atttaagatc tcccgcgtac tctgcgttga ttacactgct      660

taagggcgaa ttcgtttaaa cctgcagga                                        689


<210>  56
<211>  1364
<212>  DNA
<213>  Artificial Sequence

<220>
<223>  /note="Nukleinsäuresonde Seq.-ID Nr. 56"

<400>  56
caataacgaa ttaaccctca ctaaagggac tagtcctgca ggtttaaacg aattcgccct       60

taagcagtgg tacccataaa gagtactttt tttttttttt tttttttttt tttttgtcac      120

attaatgatt cagtaatttt attttaaata taataaaaaa gatcaccggg aacttttaat      180

tatacataaa gaacaatgta ttaatatttt gcgaaaaata gaaagttccc caacaaaaaa      240

aaaaaattgc ctataataaa aaaaaaaata aataattcct aatttaaggg atttaatgtt      300

tgtgaacttc tttctttgca tcaccagaat ttttggtgat gttgccttca gcttgcatct      360

gttgattacc gatggcttta ccagtgtttt cttttacaat tccagtagac gctatccttt      420

aatccttctg cttgtccttt tgtttgggct gctttatatt ccccttcacc ttcaagtttt      480

ttggcttttc catcggcttc catttgttca ttacctatcg ctttaccaat agtttccttg      540

ggagcacctt ggtagtattt ggtgttagca tccactctgg atggttcaga catgtttatt      600

ttttttaatg taaatgtaaa tataaaagat ttacatttat ttggtttttt ttcttgattc      660

gaatttgatt taatttgatt tccccgcgca ctctgcgttg ataccactgc ataagggcaa      720

attcgcggcc gctgacttca attcgaccta aagtgagtcg tattacaatt cactggccgt      780

cggtttacaa cgtcatgact ggtaaacccc tggcgtgacc caacttaatc gacttgcagc      840

aaatccccct ttcaccacta gactaatgac gaaaaggtcg cacccataac ccttcccata      900

gttgcgcggt ctatacctac gggagtgtaa aggttacccc tataaaaaga gaacccctaa      960

ccttcggtgg atcagtttgc acgcagtccg tggtttgcgg ggccaaggtg ttttttcccc     1020

ccgggccgtc cctttcgcgt tccaaaaaaa ctccccgaaa atttacccgg gggtgcatat     1080

cgggaaaaaa agtgggcctt gaaaaccccc ataaggccct ttttccggcc cccctttttg     1140

ggggaaaaaa tggggggatc cccccccccc cgaaaaaaac cccacaaaaa ccccccttaa     1200

ccccgggggt ttttggggaa aataaaaagt gtgggggggg gggaaaatta taaaaaaaaa     1260

gaattttttc ccccacaaat cttttttttt ctcttttaaa aaaaaaccca cccccgccca     1320

aaaaaaaaaa aaggcgcgtc tcactccacc ccaccacccc aaaa                      1364


<210>  57
<211>  1379
<212>  DNA
<213>  Artificial Sequence

<220>
<223>  /note="Nukleinsäuresonde Seq.-ID Nr. 57"

<220>
<221>  misc_feature
<222>  (1239)..(1253)
<223>  n is a, c, g, or t

<220>
<221>  misc_feature
<222>  (1259)..(1272)
<223>  n is a, c, g, or t

<220>
<221>  misc_feature
<222>  (1275)..(1278)
<223>  n is a, c, g, or t

<220>
<221>  misc_feature
<222>  (1287)..(1293)
<223>  n is a, c, g, or t

<220>
<221>  misc_feature
<222>  (1295)..(1295)
<223>  n is a, c, g, or t

<220>
<221>  misc_feature
<222>  (1298)..(1298)
<223>  n is a, c, g, or t

<400>  57
ccacaaaaga attacccctc actaaaggga ctagtcctgc aggtttaaac gaattcgccc      60

ttaagcagtg gtacccataa agagtacgcg gggaatttag tattttacaa gatgtgcaaa     120

catatactta acgctcaggt ttcgattcgt gcgcaatgct gtcaacgttg gtttgattgt     180

gccgattgcc acgcggaagt atcagatcat aaactaagta aaactcttga gatggtcttt     240

gcttgcaaaa aatgcaaaaa atgtttccgc aaggatatgg agaactttga tgaaacggat     300

gaatattgcc ctcattgtga taatcattat gtgttggatg caaagactcc gtcattcgga     360

attggagtcg agggtgaaga tccaaggaaa gataacagaa tgattaagga tgaacgttta     420

aaacaagatc ctaacaagag tattctcaat caagactttt catataaaat gggttaaata     480

attaactaca taatttgtaa ttcttttttt tttatataat acatattgcc tttagatata     540

ctaatttatt aatttgttaa tttatttaat ttattgaaat tatagaagta aaaactgaga     600

ttagtaaaaa ttagaaattt attgtcatag aatatcattt agttgataaa ttttataatt     660

aaaataaata tatttttggt gcttatttta aagaaaaaaa aaaaaaaaaa aaaaaaaaaa     720

gtactctgcg gttataccac tgcttaaagg cgatttcccg ccgcctaaat tcatttcgcc     780

taaaggggat cgtattcaat tcaactggcc ggctttttca accgcagggc tgggaaaacc     840

ctggcgttcc cccacttaat cgcctttgag caaatccccc ctttcccacg ctgggtaata     900

agcgaaaggg cccgccccca tcggcctttc aaacagttgc ccaacctcat ccttaagggg     960

ggtttaaggt ttaaccctat taaaaaaaaa gccccgtttc gtctggtttg gggatgtaca    1020

gaagggaatt tttttgaaac ccccgggggc aaccggatgg gggacccccc ctggcccagg    1080

ggcacttctg cctgtccaaa aaaagtctcc ccggggacct ttacccgggg ggtgcaaatt 1140

cggggaataa aaaccctggg cccaaaaaaa aaccccccca aaataggccc gcggggttcc 1200

cgggtccccc ctttttttcg ctatgtcaca ctgtcgagnn nnnnnnnnnn nnncgaggnn 1260

nnnnnnnnnn nnttnnnntt ttgatgnnnn nnncnccnct aggcggtggg gggggggggg 1320

gggtgggggg ggggagagaa aagaatgatg gtagtgaggg gtggggtgaa gagttaaat 1379

<210> 58
<211> 646
<212> DNA
<213> Artificial Sequence

<220>
<223> /note="Nukleinsäuresonde Seq.-ID Nr. 58"

<400> 58
attaaccctc actaaaggga ctagtcctgc aggtttaaac gaattcgccc ttaagcagtg 60

gtatcaacgc agagtacttt tttttttttt tttttttttt tttttttttg gaaaataata 120

ttttacctat cataatcata ggatcctagc ttttatctgc atatgcggtt ttccggatat 180

attatataat ttttacctat tataaaccat agaaaaaaaa atgggagatt ctatagaaag 240

cagcgaaaat tcaatcgcat ctaccccaat tagaatggca acttacgaag ggctgggacc 300

tgatatcggg gggtcttctc catcaactcc acgacgggct atatacatgc aaacaccggc 360

ggcaataaaa aaacaattac gagaccaact aaatgaaaaa tcaaaccaaa tccaaatgac 420

cgccgaatta ggacaagcac tagtaaagca acaaactgaa ctggaacaac gaatacgaga 480

acttgatcaa actcaaggcg atgaagttcc tcaagaactt aaagataaat tggccgaatt 540

agaaaaacaa gctaatgctc ttgaagcaaa tacagccaaa gtcttccttg gtactaaagg 600

tcttacttcg gggggaccag atttagccgg atctactgga tctcct 646

<210> 59
<211> 655
<212> DNA
<213> Artificial Sequence

<220>
<223> /note="Nukleinsäuresonde Seq.-ID Nr. 59"

<400> 59
attaaccctc actaaaggga ctagtcctgc aggtttaaac gaattcgccc ttaagcagtg 60

gtatcaacgc agagtacttt tttttttttt tttttttttt tttttcgtaa tattagataa 120

aaatgtcgaa ttcacttttg gccgcagcat tgagcgctgt tacgaataat acaattatta 180

cttatggtga ttatgttgga aaaaaaattg gtgataatat ttcatattca gtattggacc 240

aaaaatcact tcatcaatct catttggttg gcggtaaagt accaacatta tcttctaaag 300

atcctgaagt atatcatcct cctaaagaac ttaaaatacg tcatcaagtg tttacacccc 360

ttgaaagttt cgggagtgcc caaatattcg ttcaaacatt aactggtaaa agtattacaa 420

tatgcgttga ttttagcgat acaatttata acctaaaaga aaaaattcag gataaagaag 480

ggatacttcc agataaacaa agattagttt ttgctggtat gttacttgaa gatggccgta 540

cgttatcgga ttacaagata aaaaaacatt ccacaataca tctgatattg agactacctg 600

gtggtggacc tagtagtact acggctttat atattcaacc tgatcaactt gctcc 655

<210> 60
<211> 766
<212> DNA
<213> Artificial Sequence

<220>
<223> /note="Nukleinsäuresonde Seq.-ID Nr. 60"

<220>
<221> misc_feature
<222> (665)..(666)
<223> n is a, c, g, or t

<400> 60
ctcactaaaa ggagtagtcc tgcgggtgta aacgaatatc gcccttaagc agtgctatta 60

acgcagagta cttttttttt tttttttttt tttttttttt ataataacag gaatcattaa 120

gtcttcgttt attttacatt gcaaaaattt gtaattttaa acatcgcaac cacactaata 180

aaataatgaa tgggataata attttatgga agctgtacag acataatagc atcaaccata 240

tcgttattat gattcttcag cgcttttatt ggtttaactc gggacacatt atcttgttgc 300

atgaccaatt cgacatcctt agcctcaaca ttaaattcat ccatattcaa atcttcttca 360

tcgtcgtctt catcagcagc tgcttcatct atcttaatgg tatcttgagc agcaaaaaag 420

gccccagttt ccgcagcttg gaattgttga acaacgttag cttgagcttg agaattaaaa 480

tcctccactt tagcttctcc gaacacaatg tagcaatcag aattggggga tttataaaca 540

tcaggattaa aaatgacaag gaggatgttt tttggccttc ggatggtaac acaattgatt 600

cctggcacca ctttcaaccc tagcttctgc atttgcttgc gagccttctt ttccccgcga 660

gtctnncgtt gataccactg cttaaagggc gaattcgatg gccgctaaat tcaattcgcc 720

ctatagtgag tcgtattaca attcactggg cggctgttta caacgt 766

<210> 61
<211> 795
<212> DNA
<213> Artificial Sequence

<220>
<223> /note="Nukleinsäuresonde Seq.-ID Nr. 61"

<400> 61
acccctcact aaagggacaa gtcctgcagg tttaaacgaa ttcgccctta agcagtggta 60

tcaacgcaga gtactttttt tttttttttt tttttttttt ttttttggaa aataatattt 120

tacctatcat aatcatagga tcctagcttt tatctgcata tggagttttc cgaaaatatt 180

atataaattt tacctattat aaaccataaa aaaaaaaatg ggagattcta tagaaagcag 240

cgaaaattca atcgcatcta ccccaattag aatggcaact tacgaagggc tgggacctga 300

tatcgggggg tcttctccat caactccacg acgggctata tacatgcaaa caccggcggc 360

cataaaaaaa caattacgag accaactaaa tgaaaaatca aaccaaatcc aaatgaccgc 420

cgaattagga caagcactag taaagcaaca aactgaactg gaacaacgaa taccagaact 480

tgatcaaact caaggcgatg aagttcctca agaacttaaa gaaaaattgg ccgaattaga 540

aaaacaagct aatggtcttg aagcaataca gccaaagtct tccttggtac taaaggtctt 600

acttcgggtg gaccaaattt agccggatct actggatctc ctggtccttc tgctacatca 660

ataacagatt caacacccac aacaactacg acaggacgcc caaatagacg tgaattaaac 720

aaagcgaaaa aaacgtaaag atgatattga acttgccacc gcaataggac agggtctatt 780

actggagata cgacg 795

<210> 62
<211> 757
<212> DNA
<213> Artificial Sequence

<220>
<223> /note="Nukleinsäuresonde Seq.-ID Nr. 62"

<400> 62
ctcactaaag ggactagtcc tgcaggttta aacgaattcg cccttaagca gtggtatcaa 60

cgcagagtac gcgggcgaag aaatcgatgg ggtgttatta atgtggaaaa tgaagatcat 120

tgtgaatttg tccatcttcg agattttctc acacgaaccc accttcagga tcttattgaa 180

actactgctc aaattcatta tgaggcattc cgttcaaaac aattgttggc tttgaaagaa 240

accagtagca agcaacatga acaacagcaa caacaatcac aacctgtgca gccgcaagta 300

ggacctggtg gaccagttat ttaagaaaat agaacaacgt aaaaaaaaaa ttgaaaattc 360

aagggggtaa aggaggtaac gggtaatgtg ggttttagaa attatattgt attttcttat 420

tttagaatat atttcataaa ttacttccct tttttaactt accgtaactt tttccttgat 480

atattttgtt tgaaatttat ttatatttct tttttgttta aataattttg ttagaacttt 540

ggatatattt atacgtcatt atttttatat atttattact atcttgatat atgataagca 600

gaatgtatac tcggaattta atatttaatt ttattttgaa atccgagtga tgaaagatca 660

tcttattgtt gggaaacctt taaaattatt tatcaatact ttaatctgca aacaaatatt 720

aataatattt ttttttttca tttctttgaa ttgccct 757

<210> 63
<211> 702
<212> DNA
<213> Artificial Sequence

<220>
<223> /note="Nukleinsäuresonde Seq.-ID Nr. 63"

<400> 63
gggcgaattg aatttagcgg ccgcgaattc gcccttagcg tggtcgcggc cgaggtactt 60

tttttttttt tttttttttt ttttcaatac caacttcttt ataaagttta ataagacgtt 120

tcgctttttc aatagttgct tccttatcaa aagaaagact tgcatcaact tcagtagaaa 180

ctcttccggg aataattttc aggatttcaa ctccaaaatt aatcaataat ttatccatag 240

cccattcaac cttttcatca attgaccctt ctttgctctt tgcatattca atggctgaat 300

caatcaaatg ggcgtaattt ggtttttgcg tagcagctaa tatcaatgat ggattcgtag 360

tagcatcctg aggcttatat tgtgaaattg tttcaaaatc accagaatcc gcgactctgc 420

gttgatacca ctgcttgctc tgcgttgata ccactgctta ctctgcgttg ataccactgc 480

ttactctgcg ttgataccac tgcttactct gcgttgatac cactgcttac tctgcgttga 540

taccactgct tactctgcgt tgataccact gcttactctg cgttgatacc actgcttact 600

ctgggttgat accactgctt actctgggtt tgaaccacgg cttactctgc gttgaaacca 660

cggcttgagc aatagtactc tgcgtggaaa cccctgcttt tt 702

<210> 64
<211> 889
<212> DNA
<213> Artificial Sequence

<220>
<223> /note="Nukleinsäuresonde Seq.-ID Nr. 64"

<220>
<221> misc_feature
<222> (639)..(639)
<223> n is a, c, g, or t

<400> 64
gggcgaattg aatttagcgg ccgcgaattc gcccttagcg tggtcgcggc cgaggtacta 60

tccttggtct cgatcaaact tcttcaactt ggttcgttga tcctacagtg ccatttagta 120

aaacaggaca acttgatgcc ttaccttctg gaaccggaaa tatagtctct ttagagttta 180

actacatcta tcgatggcat ccagctgtct cagaagatga tacaaaatat gttgtggatg 240

agttcaaaac gatttttgga gatgattggg aaaatttagc tattgataaa ttcaaggaaa 300

agatgtctac ttggaagagg tcgataccca aagatccatc caaatggaag tttaataata 360

tagaaagagg aggtgataac cgtttcaaag atactgatat tgcaaaagag atcattgatg 420

gaacaagaaa agtttcaggt gcttttggag caaatcgcgt tcataaagtt ttccgtccta 480

ttgagcttct cggcattgaa tcagctagat ctttgggatt aagtagtctc aacgattttc 540

gtcgatcctt aaacttgaag ccttatgata catttaagga aatgaatcct gatccaaagg 600

tagcggaaaa actcgaagaa ctttatgaac atgtagaana tgtcgaattg tatcctggtc 660

tcatgacaga aaaaacgaag cctgctatgc tcggttcagc aattgcgctt ccatttacca 720

tcagccgcgc tattctatca gatgcgatta atcttgtacc tgccccgggc gggcgcccgg 780

gtaggtactc ttatcattgg tgctgctggt gatgctggtg gtgctgctcc aggaagtgct 840

actccaccaa cggaactgct aacatgccag caaccccatc tgatggtgc 889

<210> 65
<211> 797
<212> DNA
<213> Artificial Sequence

<220>
<223>  /note="Nukleinsäuresonde Seq.-ID Nr. 65"


<220>
<221>  misc_feature
<222>  (692)..(692)
<223>  n is a, c, g, or t

<220>
<221>  misc_feature
<222>  (726)..(726)
<223>  n is a, c, g, or t

<400>  65
aaccctcact aaagggacta gtcctgcagg tttaaacgaa ttcgccctta gcgtggtcgc      60

ggccgaggta cagccgctat tttttcaaaa gtgattcctc catcactttt gtgcgagaaa     120

gaaaatcccc ctccaacatc aagtaaatga aaatcgaatc ccatttctga tgcttgatta     180

aatacaaatc tagccctacg aatagcatca atataagagt tttcgtcata acaaccacta     240

ccaacatgaa agctaactcc aataacgttt aaacctaact cgagagcagt ctttagaaga     300

aagcccgtat catttaaaga agcaccaaat ttagctccca actttattag agctttagaa     360

tcatcagcaa gaatgcgaat gaccagttga gccctaggga ataaacgttt gatcttgtgt     420

agttcatctg cattatcaaa agttatcatt ttgacatcat gttcagcagc gaatcgtata     480

aacgacgcct gtttgcatgg attagcatag ataatgttag acggactgac acccatgtca     540

agaacagttt gaatctcatt tttgctagca caatcaaatc ctgttcctaa ggcagctaac     600

agtctgagca acataggatc tccgttgcat ttgactgcat aaaacggctc aactcgtggt     660

aaataagttt tccagcgaag atgttgacga tnaatttcgc ccaggtctgc ggcaaaaaaa     720

gcatcnatta tttcagtctc ataatttgtt gacaatattt gagatttcaa gacttcttca     780

accgataact gctaata                                                    797


<210>  66
<211>  777
<212>  DNA
<213>  Artificial Sequence

<220>
<223>  /note="Nukleinsäuresonde Seq.-ID Nr. 66"

<400>  66
gggcgaattg aatttagcgg ccgcgaattc gcccttagcg tggtcgcggc cgaggtacag      60

aagcggataa aatcaaatat cctcgagatt gtaattgtag tggagcatgt aaagaatgta     120

gtgtggagtt aaacttacac gttaaatgca ctgaagaaga tacaacgaaa gatgtgacta     180

gtaaagatct tgtcagtcac gatccgcggt ttacaccgat cactgaggga ccaaaagatg     240

cgggtatttt aattgcaaaa cttcgcaaag gtcaagaaat taaagttaaa tgtattgcta     300

agaagggagt ggcgaaggaa catgctaagt ggagtccttg tgctgcggtt gcttttgaat     360

acgatccttt taataaactt agacatacac attattggat agaggaagat gaaaaaaagg     420

aatggccaca aagtaaaaat gctaacgatg attttcctcc tttagatgat gaaatttttg     480

attttactgc gaaaccaaat agattttatt ttgaaatcga gacaattggt tctatgaaac 540

ctgaagacat tgttatgaat gcatttaaga aattgttaga aaaaatttca tcaattcaga 600

tgggattaca ttcaaatgat gttcctatta atggcagaga accaactaga gatgtttttt 660

agaatattat ttagctatga caagttccat ctacttatac catcaaaaat ttaccattag 720

atgtattatt tgagattatt tataagtatc aattatgaca agattcaact gtagtaa 777

<210> 67
<211> 766
<212> DNA
<213> Artificial Sequence

<220>
<223> /note="Nukleinsäuresonde Seq.-ID Nr. 67"

<220>
<221> misc_feature
<222> (733)..(733)
<223> n is a, c, g, or t

<400> 67
ggcgaattga atttagcggc cgcgaattcg cccttagcgt ggtcgcggcc gaggtactca 60

agatgatatc ttttaccctc atttaaccgt aaaggaaacc ctcggttaca ctgctcgatt 120

acgtcttcca cgtgaattgt cacgacgtga taaattaaaa caagtcgaaa atactatggc 180

attattaaat ttgacgaaat gtgccgatac gataataggt gaccaagcaa ttcgtggtat 240

atcgggagga gaaagaaaac gaaccaatat tgcatctgaa cttttaacag atccaagtgt 300

cattttacta gatgaaccaa catctggttt ggattcttca ttggctcttg agcctacaaa 360

aattcttaaa gaattcgctg ttaaacaaaa gaaaaccatc attatggtta tccaccaacc 420

ttcatctcaa gtatttgaat ctttcgataa attaatgtta atggctgatg gacatatggt 480

ctattttggt gaaagagctg atgtggtgga ttaccttgat gatcaaggat ataaatgtca 540

ctctaatttc aatcctgctg attatatact ggaacttttg aacgataata caatcaaaca 600

aaaattaata aatgcatatg cgcataacat tcgtgatgat ccaactggaa aacatgtagt 660

cgaaaagcat tctcgaagaa cgcgtacctg cccgggcggc cgctcgaaag ggcgaattcg 720

tttaaacctg cangactagt ccctttagtg agggttaatt ctgagc 766

<210> 68
<211> 902
<212> DNA
<213> Artificial Sequence

<220>
<223> /note="Nukleinsäuresonde Seq.-ID Nr. 68"

<400> 68
ttaccctcac taaagggact agtcctgcag gtttaaacga attcgccctt agcgtggtcg 60

cggccgaggt actttttatt ttattttatt ttattttttt tttacattct ttttttttgt 120

aatttatatt tttttttttc tctttaaaaa aacttttttc ataaaaaaaa atggacgtgg 180

aagcgagaag gaaatattgt ggactcttga tgttccctcc aacaccatca aggaacaatg 240

atattaacat taataatatg tcaatgaggg acgttgaccc ttcctcatcg acttatgaat 300

catctagaat catgatgaat gaccatcaat tcgttatgca agacggtttc ggtgaattta 360

cagcttcaag tcgcgctctc gcagctggag ccgctgctgg tggtgactta ataaatcaag 420

aaaatcttga tacttcaaat gtaatagatg gtgatgcaaa ttcaaaatct caacctgcaa 480

aaagaactcc aagacctcca aatgctttta tcctttatag aagagcaaaa caacctggta 540

tcatcgcatc tcaaagaaat ttaaccaatg ctgaagtatc aagaacaata agtgatatgt 600

ggagaaaaga acctgaagaa attcgattag aatgggaaag atacgccgat cgaaaaaaat 660

tggaacatat gcaaacttat ccaaattatg tttaccgtcc caataaaaat aagtccaaag 720

ttgacaaaaa aaggcatcga agaaaacaaa tgtctcggcc gcgaccacgc taattgcgaa 780

tttacaaccg ctaaattcaa ttcgccctat agtgagtcgg attagaatta ctggccgttc 840

gtttacaacg tcgtgactgg gaaaaccctg gcgttaccca acttaatcgc cttgcaacac 900

at 902

<210> 69
<211> 296
<212> DNA
<213> Artificial Sequence

<220>
<223> /note="Nukleinsäuresonde Seq.-ID Nr. 69"

<220>
<221> misc_feature
<222> (244)..(244)
<223> n is a, c, g, or t

<400> 69
gggactagtc ctgcaggttt aaacgaattc gcccttagcg tggtcgcgtt cgaggtactc 60

tgcgttgata ccactgctta agcaatggta cgcgggggac aacatggagc aaaggctggt 120

ataggagttt attggggtaa agatgatcca cgtaatatca gtgagcgttt gccagggccg 180

agacaaacta ataatagggc cgaaattatg gcagtaatac gcgctttaga aacttgccca 240

gacnataaca taccattaga aataaggacc gacagtcaat atgtaataaa tgcata 296

<210> 70
<211> 858
<212> DNA
<213> Artificial Sequence

<220>
<223> /note="Nukleinsäuresonde Seq.-ID Nr. 70"

<400> 70
   ctaaagggac tagtcctgca ggtttaaacg aattcgccct tagcgtggtc gcgcccttta 60
   aagcaatgaa gtgtccgacg atgatgatgt tttgaacctt tcggaatctt taaataatct 120
   tgatgatgtt gaaggaggtg accttatacc tctaccatta ggtaaacgta gaagattttc 180
   tatgggacct gattttttgg aaccactgga ttgtgatcca acatatattc cacatttaag 240
   aaaaaaacgt gcagtgcttt gtatctatgt taaattaccg ggtgaggctg cgtatcgcgc 300
   tatttattta aatcatctta ctctccagga tcttatatct aaattatctg aaaagccctc 360
   cgaaaaaatt gatctccaag gtcaacagat tacgagtgtt gtcagatgca ctaaacgtaa 420
   tctaaccgtt cgcattgatg atgattcaat tcatcaactt gaagatgaaa ctgatatgga 480
   agtagatgtt caacttaatc aaggtgggac gacagtacct cggccgcgac cacgctaagg 540
   gcgaattctc ggccgctaaa ttcaattcgc cctatagtga gtcgtattac aattcactgg 600
   ccgtcgtttt acaacgtcat gactgggaaa accctggcgt tacccaactt aatcgccttg 660
   gagcacatcc ccctttcgcc agctggcgta atagcgaaga ggcccgcact gatcgccctt 720
   cccaacagtt gcgcagccta tatcgtacgg cagtttaagg gtttacacct ataaaaagag 780
   agagccgtta tcgtctgttt gtggatgtac agagttgata tatttgacac gccggggcga 840
   acggaggaga tccccctg 858

<210> 71
<211> 973
<212> DNA
<213> Artificial Sequence

<220>
<223> /note="Nukleinsäuresonde Seq.-ID Nr. 71"

<400> 71
   gggactagtc ctgcaggttt aaacgaattc gcccttagcg tggtcgcccc cttaaaacga 60
   agtttgcgta gatattgaag tcttccgaat cccttaggta aaacggtaat tggattatcg 120
   cgaacatcca aattgacaag tgttttaatt tctccaatct ctggaggtaa ttctacaatt 180
   ttattacaat caagcaacaa agttccaagt ttcttaagag atccaataga agatggtatg 240
   tatgtcaact gattctctga taatctaaga tcaacgagct tatgtaagaa tccaattgta 300
   tctggaatat attctaattt atttttggac aggtcgagtt ttgttaaatt tttcatctat 360
   ccaatctctg ccggtatttc tgtcaactca ttacagcgta tttgtagaca attcgtaata 420
   tatagaaaac agatattttt acttaatttt actagcccaa gagaacaaag cccaatattt 480
   tttaagttag gtaattcact aaataatata tgacttagtt cgctattttc atctattaat 540
   tcatcagtta ctccatgaaa acacgaacgg tcatcaccat tatgagaatg taatatcgcc 600
   catgataatt cttcgtcagg tttatctgat tccgttacag gttcgggata aataacatca 660
   gtagctgtcg ttactatcga tgccgtttct ccacttggtg gaatcagagt aaagatgatt 720
   agatacattc tgtaaatgat tattaagttc ataaaggtca ttcaaaaatg aatctggagt 780
   attctgaggt tggatgtaac ctcgcccgcg accaccgtta agggccaatt tcccggccgc 840
   caaaattcaa attcgcccta aaagtgaagt cgtaattaaa ttccactgcc cgtcggtttt 900
   acaacgtcct agactgggaa aaaccccggc gttaacccaa cttaattccc cttgcgacaa 960
   atccccccct ttc 973

<210> 72
<211> 1005

<212>  DNA
<213>  Artificial Sequence

<220>
<223>  /note="Nukleinsäuresonde Seq.-ID Nr. 72"

<400>  72
cctcactaaa gggactagtc ctgcaggttt aaacgaattc gcccttagcg tggtcgcgcc      60

cttaagactt tttttttttt tttttttttt ttgcttattt ataggattta attgactctt     120

acaataaccc ttacaattct caatattctc aataactatc aatgcctgta ttctcaaacc     180

ccgttatccc tgcaacaacg agcaccatca acaataatag tttgccggac aaatgtatgt     240

gtgtcgatgg agtttgtcga gatccttcac acaagcacta tgctgaacaa cgtaaaaaac     300

aacaagaaca attaaggcaa caacaactgt tgcaaaagag aaagcgacgt ctttctctta     360

caacggaaat ttcatcagtt agattattac caaccacaac ttgttcactt tactccggtt     420

caaagttcag aggtgaacaa cgaagtggta gaagcagcta taacgtctcc gttcatattc     480

agcatgttga tctttcagaa tcattcttgt gcggatatct tcatattcaa gggcttaccg     540

aggactatcc tgagttgact acattctttg aggcggaaat tattggcaga aaatattcat     600

ttcttaccaa gaaatgggac gccgatgaca aagtagatat acaacattgg actcgtttcc     660

cggcttttcg cccccttaaa agtcatatga aaaatgacgg atttgtttat gactttcata     720

ataaagattt catccttatg agatggaaag aacattttct tgtacctcgg cccgcacccc     780

gccaagggcg aattcccggc cgctaaattc aattcgccct atagtgagtc gtattacaat     840

tcactggccg tccgtttaca acctcgtgac cgggaaaacc ctggggttcc ccaacttaat     900

cccctttcag ccaatccccc ctttccccac ttggggtaaa atacaaaaag gccccccacc     960

attcgccctt tccaaaaatt ggccagccca ttacgaacgg gaatt                    1005


<210>  73
<211>  907
<212>  DNA
<213>  Artificial Sequence

<220>
<223>  /note="Nukleinsäuresonde Seq.-ID Nr. 73"

<400>  73
gggcgaattg aatttagcgg ccgcgaattc gcccttagcg tggtcgcggc cgcggtacgg      60

aaccatcaga ttcaaataaa gtatattctt gttcactacc gaaccatgga tgttgatcag     120

tataagtttt cataactttt gcgcatgaat gacgatgatt ggttctattt ggtgttccat     180

cattattata acattcattt aaaaccagga tattatcacc accacggaat ggatctttaa     240

atatagcgac gggtcttaac aaaacatcgg aatcatcacc aggtgcttga ttagtagaag     300

aaccatcaaa attccattcg cttatttcag aagcatcggc aggtttaaaa tcgagagttc     360

tagtctttga acgaaggcca ctatcaccat caatccaaat atattcggct tgaactttac     420

caccttggtc gagagaaagg tattttgcaa gagtttcagt gtttgactta ttagtgttat     480

acatcttttt caaattaaaa gaaatttctt aagtaaaaaa aaattttttt tttttgagaa     540

aaaaaaagaa gaaacttttt tttcttttga gcttgggggt tgggtcgtaa attgatttaa 600

aagttgttag aaaaaaaaca aattaaacca cagaattaac tgggaaatga gtttagtttg 660

acaaaaagtt gggtttgttt aatataaaaa gaggtagttt tgattgaatt taattttaat 720

tttaaaaaaa aagatttatt gaatttgttg ttaatgaaag gaaaaaaaaa aaaaaaaaag 780

tacctgcccg ggcggccgct cgaaagggcg aattcgttta aacctgcagg actagtccct 840

ttagtgaggg ttaattctga gcttggcgga atcatggtca tagctgtttc ctgggtgaaa 900

attgtat 907

<210> 74
<211> 776
<212> DNA
<213> Artificial Sequence

<220>
<223> /note="Nukleinsäuresonde Seq.-ID Nr. 74"

<400> 74
aataaccctc actaaaggga ctagtcctgc aggtttaaac gaattcgccc ttagcgtggt 60

cgcgccctta agggattagt taacatataa ggaggtttga aaatttcgcc tcgatattct 120

gttgagaatt cgagaccctt ctgaacggat gcttgtggat tactaccggc gacaaaaacg 180

taaccatcag gtgataagat tgcaacagag tgatacattc ttgcaactgt tgatgggttc 240

attttcgcgt atgtttttcc aacaggatcg taaagaacag gattaaatgc aggattacta 300

tttacccata aaggatttgc tttgttgccc ttacggaatc ctgcaaaacc agtttgagct 360

ccatttaaaa atacaacttg accatttggt aatattacgt tgtcaggcat aacacgaggg 420

attccaccaa aatcgtccat ctcccaagca ggttgattag cgtttaaaag cattctaccg 480

caagatgctt cagcggttga agtaatttca aatgctgctt gacctccaca aaccataatt 540

tcaggtgcgt aattattact tgagaataaa ggtaacatta aagaagttcc ggtttgtggg 600

tatgtacact tttttttttt tttttttttt ttatgtatag acttacaaaa tttataaatt 660

tttatgcaac tcagttttat ttctaaagac gcgattgcat acgatgattt acaatatcac 720

caaaatctcc cattagaaca atgcggattt tctcctgtca gcagcccgat ttactt 776

<210> 75
<211> 951
<212> DNA
<213> Artificial Sequence

<220>
<223> /note="Nukleinsäuresonde Seq.-ID Nr. 75"

<400> 75
aacacccttc ctaacgggga ctagtcctgc aggtttaaac gaattcgccc ttagcgtggt 60

cgcgcccttg ggaccgttgt ttaagcagtg gtatcaacgc agagtacagc tttgggaaga 120

ttttcttcat ccttataaat tacagcagca atattttgca aatgagcttg aagagtaact 180

gcgagatcag gattttgaac agtttcacga agatttacaa ccattccaag taatgattga 240

ttcaaagtat ttacagtttc agtagctttg cttaataatt gatttgattc ttgtaattgc 300

ctaagttgat cagcagtata agattgtgta gtagttagtt gtttcttcac tctttcaacg 360

catcgatctc tcgtcgcaat agcgagttgg acaacacgaa gagtttgcgc aatctgttgt 420

tgatcatccc gaatttctct attagtttca ccattctcct caggaataat ttttttaaat 480

aagtcttcaa ttacatcaac gaaaggagcg agttgagaat cacaattcat tataaaacga 540

cttccaggtg ttttaatatt atcttcaatt gtacgaccta gttgttcttg atattgttta 600

acagttgaaa ttgtttgaat cgccggctcc tttaaagtat taataacgat cggaacttgt 660

gtacttaaat gttggacaga tgcagcggca atcttgtccg ctttttcaag ttggtcgcta 720

aacttgaaag aaacaggttt tgcaaaaacg gaaactctac gtgcactggc atcagcgaaa 780

tcgaaagtta aacggccgaa tttgtatttc ttgacagttt cgatggattc ggtgaacgca 840

ggtataccat ataatgtttc tatcaacgcc gagggcttag aatcattatt acaattattt 900

gtatcttcaa catttgaatt tacttgcgac atatttcttc tcgcttatct t 951

<210> 76
<211> 961
<212> DNA
<213> Artificial Sequence

<220>
<223> /note="Nukleinsäuresonde Seq.-ID Nr. 76"

<400> 76
cggaaataac cctcactaaa gggactagtc ctgcaggttt aaacgaattc gccctttcga 60

gcggccgccc gtgcaggtac ctgtatcttc acaaccggct ctttctccga tcacctcact 120

taatggagga aactctggaa atgagtatca tatgtctcat catattcatc aacatcaaac 180

taattctaat acgaatgcgg ccacttatca tcattctcct ccaattgaac atgctcaaca 240

accattaaat caagaagtta caaattatag taatactgta actcctgtaa gcatgccttc 300

tccacctgct agtattcctc cttctcaatc aaatttaaga tttgagattt tgctcgaggc 360

tcctactgct gctgcccaac gaactgatga aactccaatg acttacctta ataaaggtca 420

atattatggt ttatgtattc aagaccaaga taaaattgat ggtgaattta caactattat 480

taaaataatg tttcatgatg atactcaccg taaattggct tcaacttatt ggagtttttg 540

gttaacacaa caaaattctc caaagaatgc tagagctatc gatattgata aagctgcatc 600

aacaggaatt agtaatgtgg aaacaaaatc gtttgatcgc ttacaatttc gctggcatgg 660

aaagaaaggt gcaaagatct tcatacgatt tagttgtctt tccactgatt tttcacggat 720

aaagggtgta aaaggaattc ctttaagaat tcaagtggaa accaaaagtg ataatggatc 780

aactcgtacc tcggccgcga ccacgctaag ggcgaattcg cggccgctaa attcaattcg 840

ccctatagtg agtcgtatta caattcactg gccgtcgttt tacaacgtcg tgactgggaa 900

acccctggcg ttacccaact taatcgcctt gcagcacatc cccctttcgc cagctggcgt 960

a 961

<210> 77

<211>  1074
<212>  DNA
<213>  Artificial Sequence

<220>
<223>  /note="Nukleinsäuresonde Seq.-ID Nr. 77"

<400>  77
attaaccctc actaaaggga ctagtcctgc aggtttaaac gaattcgccc ttagcgtggt      60

cgcgccccta ggtacttttt tttttttttt tttgtggtca tgtcttctaa taatgaacaa     120

actattactt taccaacagg aaagactatt aaagtaaaca cgggtttatt cattaataat     180

gaattcgttg attcaattga tggaaaacgt tttgaaacca ttaatcctgc aactgaaaaa     240

gttattacta ctgtagctga agcattacct aatgatgttg ataaagctat cgatgttgct     300

actgaagctt atgttaaagt ttggagtaaa gtttccggtt atgatcgtgg tcgtttaatt     360

aataaattgg ctgacttgat ggaacgtgac attgatgagc ttgccaccat tgaatctttg     420

gataatggta aacctttttt gcttaccaaa gctgttgata ttcaactttc tattaaaacc     480

tatagatatt atgctggttt tgctgataaa attaatggag agatatttca atctgaatct     540

gataaattcg actttacaca acgtgaacct tatggtgtta ttggtgctat tattccatgg     600

aattttccat taatgatgca atctacgaaa ttggctccaa ttttagcttg tggaaatact     660

gtcgtattaa aaacttccga atttactcct ttatctgctc ttaaagtagc agctttaatt     720

aaagaagctg gttttccaaa aggtgttgta aacatcttat caggatatgg tccaacagcc     780

ggtgctgcta tggcaagtca tatgaaactc agaaaaatct cttttaccgg ttccgttcct     840

gtaggtaaat ctatccttaa agcttcagcg gaatctaatt tgaaaaaagt tacatgggaa     900

ttaggaggaa aatctccaaa tattattttt gccgattcaa atatagaaga agcagttaaa     960

tgggctcata tgggaaattt attcctcttt ccggtcaagt ttggtgagcc gggttcccgt    1020

atatacgtac ctcggccgcg acccccctaa agggggaaat tcccggcccc caaa          1074


<210>  78
<211>  956
<212>  DNA
<213>  Artificial Sequence

<220>
<223>  /note="Nukleinsäuresonde Seq.-ID Nr. 78"

<400>  78
accctcacta aagggactag tcctgcaggt ttaaacgaat tcgcccttct aatacgactc      60

actatagggc tcgagtggcc gcccgggcag gtacttcgcg aagttgttct tctgttggat     120

tttcgatttt tttaacttca attggtcttc caacaattgt tgtgatcggt ctcctgtgag     180

gtaaaacacc ataatcataa ttaaagacac cacgaccgtg aaatagaggc attgtccaac     240

cagataattt ttgtattgtc ttttggacct tccaaatttt gcttcctttt ggattatcag     300

cctggtccca tatatcgttt tctccaaaag aaaatacagg acacagacag gcaccattac     360

gaacagctaa tttaatgaaa cctaatcttc tttttaaaac taaatcataa acaccaggac     420

gcgcatttaa actctcacca gcaccaccaa ctacaattat aatagaatgt ccagggcctt     480

tctttaaaat attttcacaa gattgtcttg atactgaaac gcatccttgg gccattaaat 540

aatcacgata aagtggggta ttgaaattag atgtaagagt taataatcga catgtaattc 600

caggaaatac tttagaaaaa ttgtttgctt ctgtagcaaa atttgtccac gctccaactg 660

aaataactcc atgtgggtga tatccaaaca cgtaattttt tgatggatct aaatctgctt 720

gcttaattag ctttatcgga aagtattcag aataccattt ccagaattta gcccttcgag 780

caaactcaaa tctccttccg ccattttccg gtgcaatatc aaaaagcaca aataatagat 840

aaagcataat ccagggcaaa aagaacacta ttgtacatcg gccgcgacca cgctaagggc 900

gaattcgcgg gcgctaaatt caattcgccc tatagtgagt cgtattacat ttcact 956

<210> 79
<211> 932
<212> DNA
<213> Artificial Sequence

<220>
<223> /note="Nukleinsäuresonde Seq.-ID Nr. 79"

<400> 79
accctcacta aagggactag tcctgcaggt ttaaacgaat tcgcccttag cgtggtcgcg 60

ccctaggtac aacaaaagct agtccatatt ataattcatc taatcttagc aatactggtg 120

gtttgggttc tcttgcacaa acacgtgctg caagtcttgc tgagaaaaat ttacgtatac 180

aatctcagca attgatgatt caacaacaac aacttaaaca acaacaaatg atacttgcta 240

gagaacaatt gctaagacat catcattctg ctgaatattt gaatggcatt ggtggatacg 300

gtggtattgc tagtggacgt cgtagtcatg atttgagttc tagcatttca gatcctggtc 360

atggaatgag aagtccttta ttagaggaat ttagaaataa caagaataag aaatatgaac 420

ttaaagacat tgttggtaat attgttgaat tcagtggtga ccaacatgga tcacgattta 480

ttcaacaaaa attagaaact gcaaatagtg aagaaaaaca attagtcttt gacgaaattc 540

tccccaatgc tttgcaattg atgactgatg tttttggtaa ttatgtaatt caaaaattct 600

ttgaacacgg aaatcaaatg caaaagacga tcttggccaa acaaatggaa ggtcatgttt 660

tatcattggc attgcaaatg tatggatgtc gtgttgttca aaaggccctt gaacacgttc 720

ttactgaaca acaagctgct ttggttaaag aacttgatgg acatgtacct gcccgggcgg 780

ccgctcgaaa gggcgaattc gcggccgcta aattcaattc gccctatagt gagtcgtatt 840

acaattcact ggccgtcgtt ttacaacctc gtgactggaa aaccctggcg ttacccaact 900

taatcgcctt gcaacacatc ccctttttcg cc 932

<210> 80
<211> 901
<212> DNA
<213> Artificial Sequence

<220>
<223> /note="Nukleinsäuresonde Seq.-ID Nr. 80"

<400> 80

ggcgaattga atttagcggc cgcgaattcg cccttaagtt cttgaggaac ttcatgttga 60

tcctgatgac caaaaggcaa ctgagattaa tctcattcaa gggagacgtc cttatatgag 120

agcatttcat ttttcatggc tttcctttaa tatagctttt acgggctggt ttgcaattgc 180

tcctctttta ccttatatta tggaagatct aaaattatcc cctgaagaaa ccgaagatgg 240

aaacattgta aatgtggcct ccgctgtatt tttccgtttt cttgttggtc ctttatgtga 300

ccgttttggc ccaaaacgtg ttatggctct attgcttata ttaggttcta tacctgtggg 360

tcttagtgct ttagtacgta cccctgttgg tttaattgtt gtacgtttct ttattggtat 420

tctcggtgct acttttgttc cttgtcaatt ctggacaact caaatgtttg gtaaaaatat 480

tgttggaagt gctgatgcta tagtaggagg ttggggaaac atgggtgctg gtattactac 540

tatattactg gctttgatcg ccgctggttt tcattctgcc ggtttaagtt ctagtttatc 600

atggcgcgtt tctttaatta ttcctgctgt tctttgcttc aagagcatta gcaagggcga 660

attcgtttaa acctgcagga ctagtccctt tagtgagggt taattctgag cttggcgtaa 720

tcatggtcat agctgtttcc tgtgtgaaat tgttatccgc tcacaattcc acacaacata 780

cgagccggaa gcataaagtg taaagcctgg ggtgcctaat gagtgagcta actcacatta 840

attgcgttgc gctcactgcc cgctttccag tcgggaaacc tgtcgtgcca gctgcattaa 900

t 901

<210> 81
<211> 1532
<212> DNA
<213> Glomus intraradices

<400> 81
atggctagtc aaattattgt cgttggtggt ggtctttctg gactttctgc cgctcataca 60

gtcttggaac atggtgctaa tgttcttgtt attgataaga actctttctt tggtggtaat 120

tctaccaagg ctacctctgg tatcaacggt gctctaacca aaactcaaat agctcttggt 180

attaaagact ctgccgcttt atttcaggag gatacaacgc gctcagctcg ggatcttgct 240

cgacctgacc ttattaaagt tcttgcagga aattctgctt ctgccgtcga atggctccaa 300

gataaattca atttggacct ttctcttgtt tcacggttag gtggacactc tcaacctcgt 360

acacatcgtg gaaaggagat gtttcctggt atgacaatta catatgcttt gatggaacgt 420

ctcgaagata ttgctgaaaa ccagccaaat cgtgcacgta tcatcaagaa agcgcgtgtg 480

acaaatttga tcaaagaagg tgaagaagtt attggtgtag aatacgaaaa ggacggtcaa 540

acattaaaag aatacggtcc cgtgattttg gctactggcg gttatgcagc tgatttcacg 600

gaaaattcgt tacttaaaaa atatcgacca gatatttatg atttacctac cactaatgga 660

gatcattgta ccggagatgg gcataaaatg gttttggcta ttggcggcaa agctattgat 720

cttgaaaaag ttcaagtaca tccgactggt cttgttgatc ctaaagatcc agattctaaa 780

attaaatttt tggccgctga agcacttcga ggtgttggag gtttgttaat aaacgcagaa 840

ggaaaacgat tctgtgatga attaggacat cgtgactatg ttaccggtga aatttggaaa 900

acaaaaggcc cagttagact tgtgctcaat agtaaagcct caaaagagat tgaatggcat 960

tgcaaacatt atgctggacg tggattaatg aaaaaaatta attccggtga agaattggca 1020

aaagaaattg gcgtctccgt agcacaatta aaagcaactt ttgatgaata caatgatatc 1080

gcttctggta aaaagaaaga tccatatggg aagaaattct ttcaaaacgc gccaatgtca 1140

attaatgaca atttccacgt ttctttaatg tcaccagttt tacattacac catgggaggc 1200

gttgaagtta cccctgattc tgaagtaaaa gatgtacaag gaaaaactat tccagggctt 1260

tatgcttctg gtgaaattgc tggaggagtt cacggagcaa atcgtcttgg tggatcatct 1320

ttattaggat gtgtcgtatt tggtcgtgtt gctggagata gtgcatcacg ccatttatta 1380

caaaatttgt ccaatgcaac tgctactcgt cgacttggac aaattgctgg tcaattggct 1440

ccttatcaag ccactgttaa cgtggatcct acaaatcaaa aagtacattt ggaaatttat 1500

tggggacaac agggtggtat ttctgtcaca aa 1532

## Beschreibung

Die Erfindung betrifft ein quantitatives Verfahren zur Analyse der Anwesenheit von Mykorrhizapilzen in Proben, bevorzugt von arbuskulären Mykorrhizapilzen, insbesondere in Proben mit einem Gehalt an pflanzlichen Wurzeln und/oder an Erde, wobei die Probe und eine daraus isolierte Fraktion Hemmstoffe enthalten können, die eine zuverlässige PCR verhindern.

### Stand der Technik

Die US 5,434,048 beschreibt eine Oligonukleotidsonde mit einer für arbuskuläre Endomykorrhizapilze spezifischen Sequenz, sowie den Nachweis spezifischer DNA durch Kontaktieren der Oligonukleotidsonde mit Nukleinsäuren, die aus einer Probe isoliert sind. Die Beispiele beschreiben ausschließlich den Nachweis von pilzspezifischer DNA durch Amplifikation eines DNA-Abschnitts aus einer DNA-Probe mittels PCR, bei der die Oligonukleotidsonde als ein spezifischer Primer eingesetzt wird. Die US 5,434,048 enthält keine Beschreibung für ein Analyseverfahren mittels der spezifischen Oligonukleotidsonde, das ohne Amplifikation von DNA funktioniert. Nachweisverfahren, bei denen mittels PCR ein spezifischer Abschnitt von Gesamt-DNA vervielfältigt werden soll, wobei die DNA aus einer erdhaltigen Probe isoliert ist, sind dahingehend nachteilig, dass Hemmstoffe, die die Amplifikationsreaktion beeinträchtigen, z.B. Huminsäuren, in isolierter Gesamt-DNA enthalten sind, bzw. nur mit großem Aufwand aus solcher DNA entfernbar sind.

Bockisch et al., Nucleic Acids Research 2005, Vol. 33, Nr. 11, e101, 1-8, beschreiben die Hybridisierung einer einzelsträngigen Nukleinsäure, die an einem Ende an einen Träger gebunden ist und am gegenüberliegenden Ende eine Markierung aufweist, mit zugesetzter DNA. Die Nukleinsäure weist jeweils endständig komplementäre Abschnitte auf, so dass eine partielle intramolekulare Hybridisierung die Markierung in die Nähe des Trägers bringt. Die Detektion erfolgt dadurch, dass die Markierung auf Grund der Hybridbildung vom Träger beabstandet wird und dadurch für ein Antikörperkonjugat zugänglich wird. In Beispielreaktionen wurde eine Sonde eingesetzt, die für bakterielle RNA spezifisch war, insbesondere für bakterielle ribosomale RNA. Für den Nachweis war es erforderlich, die rRNA zu fragmentieren.

Reuter et al., Bioresour. Technol. 2009, 3343-9, beschreiben den Nachweis von DNA-Fragmenten aus Kompost mittels PCR und betonen, dass die Reinheit von DNA aus Kompostproben für die Analyse mittels PCR kritisch ist. Für die Aufreinigung von DNA aus Kompost wird die Chromatographie vorgeschlagen oder der Zusatz von Rinderserumalbumin, um die Hemmwirkung der Huminsäuren zu vermindern. Eine Alternative zur PCR-Analyse wird nicht in Erwägung gezogen.

Ning et al., Appl. Microbiol. Biotechnol. 209, 983-93, beschreiben, dass Huminsäuren wesentlich für die Hemmung der PCR und von Microarrayanalysen bei DNA aus Bodenproben verantwortlich sind. Ning et al. zitieren, dass als verbreiteter Maßstab für die Reinheit von DNA der Erfolg einer PCR mit der DNA herangezogen wird.

Broemeling et al., JALA Charlottesv Va. 2008, 40-48, beschreiben, dass in der Forensik enzymatische Nachweisverfahren, insbesondere die PCR, häufig durch Hemmstoffe aus Proben behindert werden. Als verbreitete Hemmstoffe werden Huminsäuren für Erdproben und Indigo für Jeansproben genannt. Broemeling et al. lösen das Problem dieser Hemmstoffe, die oft gemeinsam mit DNA aus Probematerial isoliert werden, durch spezielle automatisierbare Reinigungsverfahren für die DNA.

In New Phytologist 2009, 248-260, wird die Analyse von Proteinen durch Aminosäuresequenzierung beschrieben, die aus kultiviertem, wurzelfreiem Myzel und Sporen von *Glomus intraradices* isoliert wurden.

### Aufgabe der Erfindung

Der vorliegenden Erfindung stellt sich daher die Aufgabe, ein Analyseverfahren zum spezifischen Nachweis der Anwesenheit von arbuskulären Mykorrhizapilzen in einer Probe, insbesondere einer erdhaltigen Probe bereitzustellen, dass die Nachteile von Nachweisreaktionen auf Basis der PCR vermeidet.

### Allgemeine Beschreibung der Erfindung

Die Erfindung löst die vorgenannte Aufgabe durch Bereitstellen eines Verfahrens zur Analyse einer Probe, die Erde und/oder pflanzliches Wurzelgewebe enthalten kann, auf den Gehalt an Mykorrhizapilzen, vorzugsweise arbuskulären Mykorrhizapilzen, besonders bevorzugt *Glomus intraradices,* durch unmittelbare Kontaktierung von Gesamt-DNA, die aus der Probe isoliert wurde und wahlweise Hemmstoffe der PCR enthalten kann, durch Hybridisierung mit zumindest einem einzelsträngigen Oligonukleotid mit anschließender Detektion der Hybridisierung.

Das erfindungsgemäße Verfahren bietet bei einfacher Durchfiihrung, insbesondere einfacher Isolierung von DNA aus Erdproben und/oder Wurzelproben mykorrhizierter und nicht mykorrhizierter Pflanzen, eine hohe Empfindlichkeit des Nachweises von Mykorrhizapilzen, der vorzugsweise quantitativ ist. Die Robustheit des erfindungsgemäßen Verfahrens, die es auch für DNA mit einem Gehalt an Stoffen geeignet machen, die eine PCR hemmen, ergibt sich daraus, dass erfindungsgemäß Gesamt-DNA aus Erdproben und/oder Wurzelproben mykorrhizierter und nicht mykorrhizierter Pflanzen durch ein Verfahren isoliert werden kann, das die Schritte des Zellaufschlusses, insbesondere mechanisch, von in wässrigem Puffer mit einem Detergenz suspendierter Probe, Abtrennen der wässrigen Phase, Ausfällen der Gesamt-DNA aus der wässrigen Phase und Aufnehmen der gefällten Gesamt-DNA in wässriges Medium umfasst oder daraus besteht. Optional weist das Verfahren zur Isolation von Gesamt-DNA eine Extraktion der wässrigen Phase mit organischem Lösungsmittel auf und/oder die Zugabe eines Adsorptionsmittels, z.B. Kieselgel zur wässrigen Phase, das zusammen mit der Gesamt-DNA aus der wässrigen Phase ausgefällt wird. Die Gesamt-DNA kann vor der Kontaktierung mit der Oligonukleotidsonde gelagert werden, z.B. unter Kühlung und/Einfrieren, z.B. auf-70°C, -20 °C bis +5 °C.

Die Hybridisierung mit einer für den Mykorrhizapilz spezifischen Oligonukleotidsonde erfolgt mit der isolierten Gesamt-DNA, vorzugsweise unmittelbar, d.h. ohne weitere Reinigungsschritte.

Die Empfindlichkeit bzw. Nachweisgrenze des Verfahrens ist ca. 1 bis 10 ng ds-Gesamt-DNA aus Mykorrhizapilz, die in Mischung mit anderer DNA, z.B. aus Bakterien und Pflanzen vorliegt und z.B. in 100 µL wässriger Phase enthalten ist.

Erfindungsgemäß ist die Oligonukleotidsonde an einem Träger immobilisiert, und das Verfahren umfasst vorzugsweise den Schritt des Waschens des Trägers im Anschluss an die unmittelbare Hybridisierung mit der Gesamt-DNA, um ungebundene DNA im Wesentlichen zu entfernen. Das Oligonukleotid ist für zumindest einen arbuskulären Mykorrhizapilz spezifisch, insbesondere für *Glomus intraradices,* und kann z.B. ausgewählt sein aus Oligonukleotiden, die eine Sequenz entsprechend einer der Seq.-ID Nrn. 1 bis 81 aufweisen oder daraus bestehen.

Als besonderen Vorteil verwendet das erfindungsgemäße Verfahren zur Analyse nicht die spezifische oder unspezifische Vervielfältigung von DNA-Abschnitten, beispielsweise mittels Polymerase-Ketten-Reaktion (PCR), sondern enthält genau einen Hybridisierungsschritt einer einzelsträngigen Nukleinsäuresequenz mit Gesamt-DNA, die aus der Probe isoliert ist. Der spezifische Nachweisschritt, der in dem Verfahren enthalten ist, besteht daher aus der Hybridisierung zumindest eines einzelsträngigen immobilisierten Oligonukleotids mit denaturierter Gesamt-DNA.

Das einzelsträngige Oligonukleotid ist vorzugsweise einzelsträngige DNA, PNA, oder weniger bevorzugt RNA. Die einzelsträngige Nukleinsäuresequenz, vorliegend auch als einzelsträngiges Oligonukleotid und/oder als Sonde bezeichnet, ist erfindungsgemäß an einem Träger immobilisiert, beispielsweise über eine Kopplungsgruppe an seinem ersten Ende mit dem Träger verbunden, wobei das erste Ende das 5'- oder 3'-Ende der Sonde sein kann.

Das erfindungsgemäße Verfahren vermeidet dadurch, dass der spezifische Nachweisschritt auf die Hybridisierung des Oligonukleotids mit denaturierter Gesamt-DNA mit anschließendem Nachweis der Hybridisierung, insbesondere durch Detektion eines durch die Hybridisierung zugänglich gemachten, mit dem Oligonukleotid verbundenen Farbstoffs oder Haptens beschränkt ist, die Vervielfältigung von Abschnitten isolierter DNA, und vermeidet entsprechend Probleme, die in isolierter Gesamt-DNA enthaltene Hemmstoffe der DNA-Polymerase verursachen können. Entsprechend weist das erfindungsgemäße Verfahren vorzugsweise die Isolierung von Gesamt-DNA aus einer Probe mittels eines chemischen und/oder mechanischen Ausschlusses auf, und die Isolierung von Gesamt-DNA aus einer wässrigen Mischung der Probe durch Ausfällung der Gesamt-DNA aus der wässrigen Phase. Die Isolierung kann wahlweise den Schritt der Extraktion der wässrigen Probemischung mit einem organischen Lösungsmittel, insbesondere mit Phenol, vorzugsweise mit anschließender und/oder gleichzeitiger Extraktion mit Chloroform, umfassen, und/oder die Mischung der wässrigen Probemischung mit einem Adsorbenz, insbesondere mit Silicagel (z.B. Kieselgel), Ausfällung der Gesamt-DNA mit dem Adsorbenz und Waschen des Niederschlags mit wässrigem Puffer. Gefällte Gesamt-DNA wird nach Aufnehmen in wässriger Lösung und Denaturieren mit dem immobilisierten Oligonukleotid kontaktiert.

Auf solche Weise aus der Probe gewonnene Gesamt-DNA ist für das erfindungsgemäße Verfahren bevorzugt, da diese Isolierung von Gesamt-DNA einfach ist und z.B. ohne Ionenaustauschchromatographie erfolgt. Diese einfache Isolierung von Gesamt-DNA kann erfolgen, da möglicherweise in der Gesamt-DNA enthaltene Hemmstoffe für eine DNA-Polymerase beim erfindungsgemäßen Verfahren keinen wesentlichen negativen Effekt haben. Denn das erfindungsgemäße Verfahren umfasst die unmittelbare Hybridisierung eines Aliquots von Gesamt-DNA, die aus der Probe isoliert wurde, mit einer an einen Träger gebundenen Oligonukleotidsonde, d.h. ohne Amplifikation auf Basis der Gesamt-DNA. Für das erfindungsgemäße Verfahren kann daher Gesamt-DNA mit Hemmstoffen für die Aktivität von DNA-Polymerase, insbesondere Hemmstoffen für die Aktivität thermophiler DNA-Polymerase eingesetzt werden. Gesamt-DNA mit Hemmstoffen für die Aktivität von DNA-Polymerase zeichnet sich z.B. dadurch aus, dass eine Amplifikation von Abschnitten daraus oder von Abschnitten aus zugesetzter DNA mittels Taq-DNA-Polymerase im wesentlichen keine bzw. keine spezifischen Amplifikationsprodukte ergibt. Neben Huminsäuren sind z.B. Tannine und Polyphenolverbindungen Hemmstoffe, sowie Salze, wie sie z.B. aus Proben von Kaliabraumhalden stammen.

Die isolierte Gesamt-DNA wird erfindungsgemäß denaturiert, bevor sie mit der einzelsträngigen, an einem Träger immobilisierten Oligonukleotidsonde kontaktiert wird. Vorzugsweise wird die Gesamt - DNA durch Erwärmen auf 95 - 100 °C für 1 bis 5 min und anschließendes schnelles Abkühlen auf 0 - 5 °C denaturiert.

Erfindungsgemäß wird ein Signal, das durch die Hybridisierung der Sonde mit einem Anteil der mit dieser kontaktierten Gesamt-DNA erzeugt wird, detektiert. Für die Detektion eines Signals, das durch die Hybridisierung der Oligonukleotidsonde mit einem Anteil der isolierten Gesamt-DNA erzeugt wird, ist das Messen eines Farbstoffs bevorzugt, der mit der Oligonukleotidsonde verbunden ist, oder das Messen eines Farbstoffs, der durch Umsetzung eines Substrats durch ein mit der Oligonukleotidsonde verbundenes Enzym erzeugt wird.

In Ausfiihrungsformen, in denen die Oligonukleotidsonde mit einem Farbstoff verbunden ist, ist es bevorzugt, dass ein Farbstoff, vorzugsweise ein Fluoreszenzfarbstoff, am zweiten Ende der Sonde angebunden ist, während ein Quencher, der vorzugsweise auf den Farbstoff abgestimmt ist, am gegenüberliegenden ersten Ende der Sonde angebunden ist. Vorzugsweise weist die Oligonukleotidsonde zusätzlich zu oder als Bestandteil der für einen arbuskulären Mykorrhizapilz spezifischen Nukleinsäuresequenz an ihrem ersten und zweiten Ende jeweils einen komplementären Abschnitt auf, sodass die Sonde mittels ihrer endständigen komplementären Abschnitte partiell unter Ausbildung einer Schleife mit sich selbst hybridisiert.

Für die Detektion kann die Oligonukleotidsonde an ihrem zweiten Ende mit einem Hapten gekoppelt sein, das in einer Hapten-spezifischen Nachweisreaktion, beispielsweise mit einem Enzym-gekoppelten Antikörper nachweisbar ist, wobei das mit Antikörper gekoppelte Enzym ein Substrat zu einem Farbstoff umsetzen kann, der als Signal für die Anwesenheit von Mykorrhizapilz-DNA gemessen wird.

Besonders bevorzugt ist die Oligonukleotidsonde mit ihrem ersten Ende an einen Träger angebunden, beispielsweise über ein Paar miteinander assoziierender Kopplungspartner, von denen der eine am ersten Ende der Oligonukleotidsonde angebunden ist und der zweite am Träger. Paare solcher Kopplungspartner sind beispielsweise Biotin und Avidin bzw. Streptavidin, ein Hapten und ein für das Hapten spezifischer Antikörper, sowie kovalente Bindungen (z.B. mittels wasserlöslichem Carbodiimid oder N-Hydroxysuccimid hergestellt), Adsorption und Thiolgruppen zur Ausbildung von Disulfidbrücken.

Aufgrund der Immobilisierung der Sonde an einem Träger ermöglicht das erfindungsgemäße Verfahren den Schritt, ungebundene Nukleinsäuren aus der Probe durch Waschen von der Sonde zu entfernen. Das Entfernen ungebundener DNA durch Waschen im Anschluss an das Kontaktieren der Gesamt-DNA mit der an den Träger immobilisierten Oligonukleotidsonde erhöht die Empfindlichkeit des erfindungsgemäßen Analyseverfahrens.

Das erfindungsgemäße Verfahren ist daher ohne Amplifikation von DNA und weist als spezifischen Nachweisschritt allein die unmittelbare Hybridisierung eines an einem Träger immobilisierten Oligonukleotids an die aus der Probe extrahierten Gesamt-DNA auf, welche Hemmstoffe für DNA-Polymerasen, z.B. Huminsäure enthalten kann.

Im Anschluss an die Hybridisierung einer einzelsträngigen Oligonukleotidsonde mit der aus der Probe isolierten Gesamt-DNA wird erfindungsgemäß die Bildung eines Hybrids aus einzelsträngiger Oligonukleotidsonde und einem Abschnitt der Gesamt-DNA detektiert. Besonders bevorzugt wird die Bildung eines Hybrids des Oligonukleotids mit einem Abschnitt der Gesamt-DNA dadurch detektiert, dass das Oligonukleotid an seinen beiden gegenüberliegenden Enden jeweils komplementäre, besonders bevorzugt revers komplementäre Abschnitte aufweist, die jeweils z.B. 4 bis 10, vorzugsweise 6 Nukleotide umfassen, sodass bei Ausbildung eines Hybrids des einzelsträngigen Oligonukleotids mit Gesamt-DNA die Konformation der Oligonukleotidsonde geändert wird, insbesondere durch Aufhebung der Hybridisierung zueinander komplementärer, endständiger Abschnitte der Oligonukleotidsonde. Ein am zweiten Ende der Oligonukleotidsonde angebundenes Hapten oder Farbstoffmolekül wird in dieser Ausführungsform durch die Hybridbildung in einen weiteren Abstand zum ersten Ende der Oligonukleotidsonde gebracht, mit dem die Oligonukleotidsonde an den Träger angebunden ist. Anschließend kann das Hapten, bzw. Farbstoffmolekül detektiert werden, entweder unmittelbar durch optische Detektion, oder durch immunologischen und/oder kolorimetrischen Nachweis des Haptens, beispielsweise durch Reaktion des Haptens mit einem enzymgekoppelten Antikörper, gefolgt von der enzymatischen Umsetzung eines Substrats durch das an das Hapten gebundene Antikörper-Enzym-Konjugat.

Alternativ kann die unmittelbare Hybridbildung des an einen Träger gebundenen Oligonukleotids mit einem Abschnitt der aus der Probe isolierten Gesamt-DNA durch Messung der Änderung physikalischer Eigenschaften des gebundenen Oligonukleotids erfolgen, beispielsweise durch Oberflächenplasmonresonanzmessung an der Trägeroberfläche, an welche die Oligonukleotidsonde gebunden ist. Alternativ kann zur Messung die Änderung der Schwingungsfrequenz eines Trägers erfolgen, wenn der Träger einen Piezokristall aufweist oder aus Piezokristall besteht.

Erfindungsgemäß ist bevorzugt, die aus einer Probe isolierte Gesamt-DNA in einem ersten Aliquot ohne Dotierung zu analysieren, und in einem zweiten Aliquot mit einer ersten Dotierung, und weiter bevorzugt zusätzlich in einem zweiten Aliquot mit einer zweiten Dotierung, wobei zur Dotierung jeweils ein einsträngiges oder doppelsträngiges DNA-Fragment zugesetzt wird, z.B. in einer Konzentration von ca. 0,1 bis 10 nM, vorzugsweise 1 nM. Bei Dotierung von zumindest einem Aliquot, vorzugsweise bei jeweils unterschiedlicher Dotierung zweier Aliquots der Gesamt-DNA, umfasst das erfindungsgemäße Verfahren die Erzeugung einer Kalibrierkurve aus den Ergebnissen der Messungen für die dotierten Aliquots, und den Vergleich des Messwerts des undotierten Aliquots mit der Kalibrierkurve. Als Nachweis für die Anwesenheit eines arbuskulären Mykorrhizapilzes wird ein Messwert bei der Detektion des nicht dotierten Aliquots angesehen, der signifikant unterhalb der Kalibrierkurve liegt, z.B. maximal um den Faktor 2 bis 5 unterhalb der Kalibrierkurve liegt, die aus den Messwerten bestimmt ist, die für die dotierten Aliquots gemessen wurden.

Zusätzlich oder in Alternative zu der Dotierung der Gesamt-DNA in zumindest einem Aliquot, das parallel zur undotierten Gesamt-DNA mit dem erfindungsgemäßen Verfahren analysiert wird, wird vorzugsweise Gesamt-DNA der Probe in einer ersten und einer zweiten Konzentration in dem erfindungsgemäßen Verfahren analysiert. Vorzugsweise ist die zweite Konzentration etwa 2-10 mal, insbesondere 3 - 5 mal so hoch wie die erste.

Bei der Analyse von Aliquots einer Gesamt-DNA aus einer Probe in zwei oder mehr Konzentrationen werden Messwerte bei der Detektion dann als Nachweis für die Anwesenheit arbuskulärer Mykorrhizapilze angesehen, wenn die Messwerte etwa im Verhältnis der Konzentrationen der Gesamt - DNA in den Aliquots bestimmt wurden.

### Genaue Beschreibung

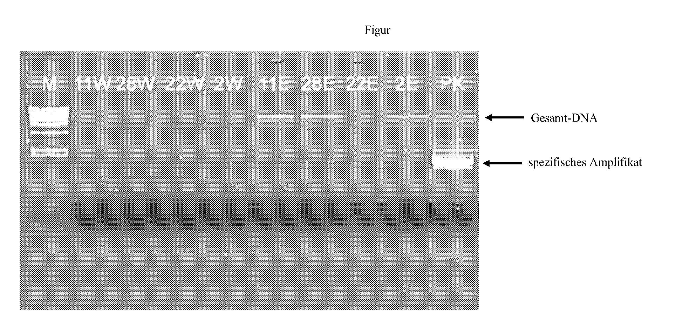

Die Erfindung wird nun genauer anhand von Beispielen mit Bezug auf die Figur beschrieben.

Die Figur zeigt ein Photo einer gelelektrophoretischen Auftrennung von PCR-Reaktionen, die als Vergleich mit Gesamt-DNA durchgeführt wurden.

### Beispiel 1: Isolierung von DNA aus Erd- und Wurzelproben mykorrhizierter und nichtmykorrhizierter Pflanzen

Da das erfindungsgemäße Analyseverfahren gegenüber Hemmstoffen, die eine PCR-Reaktion beeinträchtigen, unempfindlich ist, konnte Gesamt-DNA aus einer Probe, die auch Erde enthielt, mittels Homogenisieren und Extraktion mit Lösungsmittel isoliert werden. Die Extraktion von Gesamt-DNA wurde durch Homogenisieren und Aufschließen der Probe in wässrigem Puffer und anschließendem Ausfällen der DNA aus der abgetrennten wässrigen Phase durchgeführt. Im einzelnen wurden ca. 0,5 g Bodenprobe mit 500 µL 100 mM Na-Phosphatpuffer (pH 8,0) gemischt. Zu dieser Suspension wurden Glaskugeln und 250 µL 10% Na-Dodecylsulfat zugegeben und die Probe durch schnelles radiales Mischen mittels eines Vortex-Mixers 6-mal für 1 min aufgeschlossen, jeweils mit Kühlen auf Eis für 1 min. Die wässrige Phase wird durch Zentrifugation (Tischzentrifuge, 5.000 x g, 5 min) vom Niederschlag abgetrennt. Optional kann die wässrige Phase mittels Extraktion mit dem gleichen Volumen Phenol (25 : 1 Phenol : Chloroform), Abtrennen der organischen Phase, anschließendes Extrahieren der verbleibenden wässrigen Phase mittels Chloroform und Abtrennen der organischen Phase gereinigt werden. Aus der wässrigen Phase wird Gesamt-DNA durch Fällung abgetrennt, z.B. durch Zugabe von 1/3 Volumen Na-Acetat (3M, pH 6,5) und 2 bis 3 Volumen Ethanol oder Isopropanol, bezogen auf das Volumen der wässrigen Phase.

Bevorzugt wird die wässrige Phase vor der Fällung mit Silicagel und NaJ versetzt und unter Mischen inkubiert, die Gesamt-DNA wird mit dem Silicagel pelletiert und, vorzugsweise nach Waschen des Pellets mit wässrigem und/oder alkoholischer Lösung, in wässrigem Puffer resuspendiert. Für diese Behandlung werden zu 500 µL wässriger Phase ca. 1 mL 6 M NaJ und 150 µL 100 % Silicagel zugesetzt und die Mischung für 1 bis 10 min bei Raumtemperatur inkubiert; anschließend wird die Gesamt-DNA aus der wässrigen Phase mit dem Silicagel durch Zentrifugation (Tischzentrifuge, 1 min bei 5.000 x g) pelletiert. Das Pellet kann gewaschen werden, z.B. mit 500 µL eiskaltem Ethanol. Ausgefällte Gesamt-DNA wird in wässrigem Puffer resuspendiert, z.B. TE (10 mM Tris-HCl, 1mM EDTA, pH 6,5).

Aliquots von Gesamt-DNA, die nach diesem Verfahren einschließlich Aufreinigung mittels Kieselgel, jedoch ohne Phenolextraktion aus verschiedenen Proben gewonnen wurden, wurden als Vergleichstest für eine PCR unter jeweils gleichen Bedingungen eingesetzt. Aliquots der PCR-Ansätze wurden gelelektrophoretisch aufgetrennt; ein Photo ist in der Figur gezeigt. Die eingesetzten Proben enthielten nachweislich *Glomus intraradices,* als Arbuskeln in Wurzelproben und als z.T. gekeimte Sporen in Erdproben. Die PCR-Produkte aus Proben aus Erde mit Wurzelgewebe sind in Spuren 11 W, 28W und 22W gezeigt, die PCR-Produkte aus Erdproben ohne sichtbares Wurzelgewebe in Spuren 11E, 28E, 22E und 2E; Spur PK zeigt die Positivkontrolle, bei deren PCR per Ionenaustausch aufgereinigtes Amplifikat von DNA aus *Glomus intraradices* eingesetzt wurde, welches einen Abschnitt umfasste, für den die eingesetzten Primer spezifisch sind. M zeigt λ/HindIII restringierte DNA.

Es wird deutlich, dass mit der isolierten Gesamt-DNA keine PCR ablief, und entsprechend kein Nachweis von Mykorrizapilzen aus der Gesamt-DNA möglich ist. Dieses Ergebnis entspricht dem Stand der Technik insofern, als Gesamt-DNA aus Probenmaterial, das pflanzliches Wurzelgewebe und/oder Erde enthält, nicht auf einfache Weise und ohne erheblichen Aufwand, z.B. nicht ohne Ionenaustauschchromatographie in einer Reinheit isoliert wird, die frei von Hemmstoffen für die DNA-Polymerase, z.B. thermostabile DNA-Polymerase ist.

### Beispiel 2: Hybridisierung von Gesamt-DNA mit immobilisiertem Oligonukleotid

Nach Beispiel 1 erhaltene Gesamt-DNA wurde durch Erwärmen auf 98 °C für 5 min und anschließendes Abkühlen im Eisbad denaturiert.

Eine einzelsträngige Oligonukleotidsonde mit der Seq.-ID Nr. 52 oder Seq.-ID Nr. 19 wurde so synthetisiert, dass jeweils endständig 6 zueinander komplementäre Nukleinsäuren angefügt waren, die zueinander komplementäre Nukleinsäuren waren. An das 5' -Ende des Oligonukleotids, das als zweites Ende gewählt wurde, wurde Digoxygenin (DIG) synthetisiert; an das als erstes Ende verwendete 3'-Ende wurde Biotin synthetisiert.

Eine mit Streptavidin beschichtete Mikrotiterplatte mit 96 Näpfen wurde durch Kontaktieren mit dem mit Biotin verbundenen Oligonukleotid in wässriger Pufferlösung kontaktiert. Ungebundene Oligonukleotide wurden durch Waschen mit Puffer entfernt.

Als Kontrolltest wurden einige Näpfe der beschichteten Platte mit einer einzelsträngigen Oligonukleotidsonde (Seq. ID Nr. 52 oder Nr. 19) beschichtet, ungebundene Sonde durch Waschen entfernt, und mit anti-DIG-Antikörper, der mit alkalischer Phosphatase gekoppelt war, inkubiert und gewaschen. Für die Nachweisreaktion wurden 100 µL anti-DIG-Antikörper-alkalische Phosphatase-Konjugat (1 : 1000 verdünnt in eiskaltem 5 x SSC Puffer) hinzugegeben, für 10 min bei 4 °C inkubiert, und anschließend dreifach mit 400 µL eiskaltem 0,5 x SSC-Tween20-Puffer gewaschen. Zur Detektion wurden 100 µL des Substrats der alkalischen Phosphatase, p-Nitrophenylphosphat in Lösung zugegeben, und nach Inkubation bei 405 nm gemessen.

Dieser Kontrolltest ergab im Wesentlichen kein Signal und zeigt, dass die Oligonukleotidsonde, die jeweils endständig komplementäre Sequenzabschnitte aufweist, bei Immobilisierung mit einem ersten Ende an einen Träger eine am gegenüberliegenden zweiten Ende des Oligonukleotids angebundene Hapten- (DIG) oder Farbstoffgruppe in einem Zustand aufweist, in dem eine Detektion im wesentlichen nicht erfolgt.

Zu anderen Näpfen der Mikrotiterplatte, an denen mit ihrem ersten Ende angebundene Oligonukleotide immobilisiert waren, wurde denaturierte Gesamt-DNA in 5 x SSC-Puffer (100 µL) gegeben. Die Hybridisierung erfolgte für 0,5 - 2 h, vorzugsweise für 1 - 2 h bei einer Temperatur von ca. 22 - 50 °C, vorzugsweise bei 37 °C.

Für die Hybridisierung wurde eine Zeitdauer von 60 - 120 min, vorzugsweise ca. 90 min als geeignet gefunden, als Temperatur für die Hybridisierung 30 - 50 °C, vorzugsweise 35 - 40 °C, besonders bevorzugt 37 °C.

Für die Farbentwicklung wurde gefunden, dass bei alkalischer Phosphatase eine Inkubation mit ihrem Substrat für 1 - 3 h, vorzugsweise etwa 2 h bei Raumtemperatur ausreicht.

Für die Empfindlichkeit des erfindungsgemäßen Analyseverfahrens wurde die minimale Konzentration zu ca. 100 pg/µL Gesamt-DNA bestimmt. Vorzugsweise wird Gesamt-DNA in einer Konzentration von ca. 0,5 - 2,5 ng/µL eingesetzt. Das erfindungsgemäße Analyseverfahren zeigte eine etwa lineare Abhängigkeit zwischen dem detektierten Signal für die Hybridisierung der immobilisierten Oligonukleotidsonde mit denaturierter Gesamt-DNA in einem Bereich von ca. 0,5 bis 5 ng/µL Gesamt-DNA, insbesondere von 0,5 bis 2,5 ng/µL Gesamt-DNA.

## Patentansprüche

1. Verfahren zur Analyse einer Probe, die pflanzliches Wurzelgewebe aufweist, auf den Gehalt an Mykorrhizapilz mit den Schritten
Isolierung von Gesamt-DNA aus der Probe,
Denaturieren der Gesamt-DNA,
wobei Kontaktieren der Gesamt-DNA mit einem einzelsträngigen Oligonukleotid,
die denaturierte isolierte Gesamt-DNA unverändert mit dem einzelsträngigen Oligonukleotid kontaktiert wird,
das einzelsträngige Oligonukleotid durch Bindung eines ersten seiner zwei Enden an einem Träger immobilisiert ist,
nach Kontaktieren der Gesamt-DNA mit dem immobilisierten Oligonukleotid ungebundene DNA durch Waschen vom Träger entfernt wird, und
mit Detektion der Intensität eines Signals, das durch Bindung eines Teils der Gesamt-DNA an das immobilisierte Oligonukleotid verursacht ist, **dadurch gekennzeichnet,**
**dass**
das Oligonukleotid zusätzlich an seinen gegenüberliegenden Enden jeweils zueinander komplementäre Sequenzabschnitte aufweist und das Oligonukleotid an seinem dem ersten Ende gegenüberliegenden zweiten Ende eine detektierbare Markierung aufweist.

2. Verfahren nach Anspruch 1, **dadurch gekennzeichnet, dass** die Probe zusätzlich oder alternativ zu pflanzlichem Wurzelgewebe Erde aufweist.

3. Verfahren nach einem der voranstehenden Ansprüche, **dadurch gekennzeichnet, dass** die isolierte Gesamt-DNA Hemmstoffe enthält, die die Aktivität von DNA-Polymerase hemmen.

4. Verfahren nach einem der voranstehenden Ansprüche, **dadurch gekennzeichnet, dass** der Mykorrhizapilz ein arbuskulärer Mykorrhizapilz ist.

5. Verfahren nach einem der voranstehenden Ansprüche, **dadurch gekennzeichnet, dass** die Gesamt-DNA isoliert wird durch Suspendieren der Probe in wässrigem tensidhaltigem Puffer, Mahlen der suspendierten Probe, Abtrennen einer wässrigen Phase von Feststoffen und Ausfällen von Gesamt-DNA aus der abgetrennten wässrigen Phase.

6. Verfahren nach Anspruch 5, **dadurch gekennzeichnet, dass** der wässrige Puffer Phosphatpuffer ist.

7. Verfahren nach Anspruch 5 oder 6, **dadurch gekennzeichnet, dass** der Puffer als Tensid Natriumdodecylsulfat enthält.

8. Verfahren nach einem der Ansprüche 5 bis 7, **dadurch gekennzeichnet, dass** die abgetrennte wässrige Phase vor dem Ausfällen mit einem 1,5- bis 3-fachen Volumen wässriger NaJ-Lösung und Silicagel versetzt wird, inkubiert wird, und das Ausfällen durch Zentrifugation erfolgt.

9. Verfahren nach einem der Ansprüche 5 bis 8, **dadurch gekennzeichnet, dass** die ausgefällte Gesamt-DNA vor der Resuspendierung mit wässrigem Puffer gewaschen wird.

10. Verfahren nach einem der voranstehenden Ansprüche, **dadurch gekennzeichnet, dass** die Bindung des Oligonukleotids an den Träger durch ein Paar von Bindungsmolekülen erfolgt, von denen ein Bindungspartner am ersten Ende des Oligonukleotids gebunden ist und der andere Bindungspartner am Träger gebunden ist.

11. Verfahren nach einem der voranstehenden Ansprüche, **dadurch gekennzeichnet, dass** die Markierung ein Farbstoff ist.

12. Verfahren nach einem der Ansprüche 1 bis 10 ist, **dadurch gekennzeichnet, dass** die Markierung ein Hapten ist, ein zugesetzter Enzym-gekoppelter Antikörpers an das Hapten bindet, und die Detektion nach enzymatischer Umsetzen eines Substrats für das Enzym zu einem Farbstoff erfolgt.

13. Verfahren nach Anspruch 12, **dadurch gekennzeichnet, dass** die Markierung ein Hapten ist, und die Detektion nach Zusetzen eines für das Hapten spezifischen Antikörpers, der ein gekoppeltes Enzym aufweist, Entfernen ungebundenen Antikörpers und enzymatisches Umsetzen eines zugesetzten Substrats zu einem Farbstoff erfolgt.

14. Verfahren nach einem der Ansprüche 1 bis 10, **dadurch gekennzeichnet, dass** die Detektion durch Messung der Änderung der Oberflächenplasmonresonanz des Trägers erfolgt, oder durch Messen der Änderung der Schwingungsfrequenz eines Trägers erfolgt, der einen Piezokristall aufweist oder aus Piezokristall besteht.

15. Verfahren nach einem der voranstehenden Ansprüche, **dadurch gekennzeichnet**, die Gesamt-DNA in zumindest 2 Aliquots mit jeweils unterschiedlicher Konzentration an Gesamt-DNA in getrennten parallelen Ansätzen mit dem Oligonukleotid kontaktiert wird.

16. Verfahren nach einem der voranstehenden Ansprüche, **dadurch gekennzeichnet, dass** zumindest zwei Aliquots der Gesamt-DNA in unterschiedlichen Mengen mit DNA-Fragment dotiert werden, das einen zum Oligonukleotid komplementären Abschnitt enthält.

17. Verfahren nach einem der voranstehenden Ansprüche, **dadurch gekennzeichnet, dass** das Oligonukleotid eine Nukleinsäuresequenz zumindest einer der Seq.-ID Nrn. 1 bis 81 enthält.
